# EUROPÄISCHE PATENTSCHRIFT

(11) **EP 0 548 764 B1**
(45) Veröffentlichungstag und Bekanntmachung des Hinweises auf die Patenterteilung: **28.08.1996**
(21) Anmeldenummer: 92121357.5
(22) Anmeldetag: 16.12.1992
(51) Int. Cl.: C07C 69/734, C07C 67/343

(54) **Oxadimethacryl-Verbindungen und Verfahren zu ihrer Herstellung**
Oxadimethacrylic compounds and process for their preparation
Composés oxadiméthacryliques et leur procédé de préparation

(30) Priorität: 24.12.1991 DE 4142909
(43) Veröffentlichungstag der Anmeldung: 30.06.1993
(73) Patentinhaber: BASF Aktiengesellschaft, 67063 Ludwigshafen (DE)
(72) Erfinder: Besecke, Siegmund, Dr., W-3250 Hameln (DE); Deckers, Andreas, Dr., W-6700 Ludwigshafen (DE); Lauke, Harald, Dr., W-6800 Mannheim 1 (DE)

(56) Entgegenhaltungen:
- US-A- 4 889 948
- MACROMOLECULES Bd. 24, Nr. 8, 1991, EASTON US Seiten 2043 - 2047 R.F.COLLETTI ET AL. 'MECHANISM STUDY OF THE BASE-CATALYZED ETHER FORMATION INVOLVING ALPHA- (HYDROXYMETHYL)ACRYLATES'
- 'CHEMICAL ABSTRACTS SERVICE.REGISTRY HANDBOOK - NUMBER SECTION. 1989 SUPPLEMENT' 1989 , THE AMERICAN CHEMICAL SOCIETY , COLUMBUS,OHIO.US

## Beschreibung

Die vorliegende Erfindung betrifft Oxadimethacrylverbindungen der allgemeinen Formel I

CH₂=C(A)CH₂-O-CH₂C(B)=CH₂ I

in der A und B ausgewählt sind aus der Gruppe aus -COOR¹, -COR¹, - CONR²R³ und -CN, mit der Maßgabe, daß A ≠ B ist, und R¹, R² und R³ folgende Bedeutung haben:
- R¹ =: H, C₁-C₁₈-Alkyl, C₃-C₈-Cycloalkyl, C₃-C₈-Cycloalkyl-C₁-C₅-alkyl, wobei die Cycloalkyl-Ringe bis zu dreifach mit C₁-C₄-Alkyl- oder C₁-C₄-Alkoxy-Gruppen substituiert sein können, Hydroxy-C₁-C₅-alkyl, Amino-C₁-C₅-alkyl, N-C₁-C₄-Alkyl-amino-C₁-C₅-alkyl, N,N-Di-(C₁-C₄-Alkyl)-amino-C₁-C₅-alkyl, C₆-C₁₈-Aryl, C₆-C₁₈-Aryl-C₁-C₄-alkyl, wobei die Arylgruppen bis zu drei der folgenden Gruppen tragen können: Halogen, C₁-C₂₂-Alkyl, C₁-C₄-Alkoxy, Carboxy, C₁-C₄-Alkoxycarbonyl, Aminocarbonyl, C₁-C₄-Alkylaminocarbonyl, Di-(C₁-C₄-Alkyl)aminocarbonyl, Nitrilo, Nitro, Amino, C₁-C₄-Alkylamino, Di-(C₁-C₄-Alkyl)amino;
- R²,R³ =: H, C₁-C₁₈-Alkyl, C₃-C₈-Cycloalkyl, C₃-C₈-Cycloalkyl-C₁-C₅-alkyl, wobei die Cycloalkyl-Ringe bis zu dreifach mit C₁-C₄-Alkyl- oder C₁-C₄-Alkoxy-Gruppen substituiert sein können, C₆-C₁₈-Aryl, C₆-C₁₈-Aryl-C₁-C₄-alkyl, wobei die Arylgruppen bis zu drei der folgenden Gruppen tragen können: Halogen, C₁-C₂₂-Alkyl, C₁-C₄-Alkoxy, Carboxy, C₁-C₄-Alkoxycarbonyl, Aminocarbonyl, C₁-C₄-Alkylaminocarbonyl, Di-(C₁-C₄-Alkyl)aminocarbonyl, Nitrilo, Nitro, Amino, C₁-C₄-Alkylamino, Di-(C₁-C₄-Alkyl)amino, ausgenommen Verbindungen I, in denen A -COO-n-Butyl bedeutet, wenn B für -COO-C₁- bis C₂-Alkyl steht und Verbindungen I, in denen A für -COO-Methyl steht, wenn B -COO-Ethyl bedeutet.

Weiterhin betrifft die Erfindung Verfahren zur Herstellung der Oxadimethacrylverbindungen I sowie die Verwendung dieser Verbindungen zur Herstellung von Polymeren.

Symmetrische Oxadimethacrylverbindungen, d.h. solche, in denen A = B ist, sind bekannt. So sind aus der US-A 4,889,948 und aus Polymer Preprints, American Chemical Society, Division of Polymer Chemistry 31(1) (1990) 503 Oxadimethacrylverbindungen der allgemeinen Formel I'''

CH₂=C(COOZ)CH₂-O-CH₂C(COOZ)=CH₂ I'''

in der Z = H, Me, Et, n-Butyl, i-Butyl, tert.-Butyl, Neopentyl, Benzyl, Phenethyl, Trimethylcyclohexyl und Tetrahydrofurfuryl bedeutet, beschrieben.

Verbindungen dieses Typs sind wegen ihrer Bifunktionalität als Monomerbausteine geschätzte Verbindungen, die vielfach Verwendung finden, beispielsweise als Monomere zur Herstellung von Homopolymeren, oder als Comonomere oder Vernetzer. Bislang stehen aber nur einige wenige, und zwar ausschließlich symmetrische Oxadimethacrylverbindungen in nicht ausreichender Menge zur Verfügung. Ein weiterer Nachteil besteht in der meist nicht voll zufriedenstellenden Reinheit dieser Verbindungen.

Die Oxadimethacrylverbindungen I''' kann man nach der US-A 4,889,948 ausgehend von Alkoholen des Typs Alkohol III, H₂C=C(A)CH₂OH, als auch ausgehend von der Acrylverbindungen des Typs Acrylverbindung II, H₂C=C(A)H, erhalten. Die Reaktion der Alkohole des Typs Alkohol III zu den Oxadimethacrylverbindungen I''' wird dabei unter Erhitzen durchgeführt, wobei bei ein- bis zweitägigen Reaktionszeiten neben beträchtlicher Polymerisation der Monomeren nur mäßige Ausbeuten an den Oxadimethacrylverbindungen I''' erhalten werden.

Ausgehend von den Acrylverbindungen des Typs Acrylverbindung II, die man in Gegenwart des tertiären Amins 1,4-Diazabicyclo-[2.2.2]-octan (DABCO®) mit Formaldehyd umsetzt, erhält man als Hauptprodukt nach der US-A 4,889,948 Alkohole des Typs Alkohol III. Die Oxadimethacrylverbindung I''' entsteht dabei nur in geringen Mengen. Daneben entstehen höhere (Ether-)Homologe und polymere Nebenprodukte. Neben dieser unspezifischen Reaktion sind weiter nachteilig die langen Reaktionszeiten (10 bis 20 Tage) sowie eine beträchtliche Polymerbildung bei Temperaturen oberhalb der Raumtemperatur.

Aus Macromolecules, 1987, 20, 2039 war die Oxadimethylacrylverbindung I''', in der Z eine Methylgrupe bedeutet, sowie deren Ethyl- und n-Butylanaloga bekannt. Diese Verbindungen werden durch Umsetzen von CH₂=C(A)H mit Formaldehyd in Gegenwart von DABCO® hergestellt.

Zwei unsymmetrisch substituierte Oxadimethacrylverbindungen (A = -COO-n-Butyl oder -COO Methyl und B = -COO Ethyl) sind in Chemical Abstract unter den Nummern 118363-09-0 und 118363-07-8 registriert.

Aufgabe der vorliegenden Erfindung war es, neue Oxadimethacrylverbindungen in hoher Reinheit zur Verfügung zu stellen.

Demgemäß wurden die eingangs definierten Oxadimethacrylverbindungen I gefunden.

Außerdem wurden Verfahren zur Herstellung dieser Verbindungen sowie deren Verwendung zur Herstellung von Polymeren gefunden.

Als Substituenten R¹, R², R³ in den erfindungsgemäßen Oxadimethacrylverbindungen I kommen bevorzugt folgende Reste in Betracht:
- R¹: Wasserstoff;
C₁-C₁₈-Alkyl, darunter vorzugsweise C₁-C₁₂-Alkyl wie Methyl, Ethyl, n-Propyl, i-Propyl, n-Butyl, sek.-Butyl, i-Butyl, tert.-Butyl, n-Pentyl, i-Pentyl, sek.-Pentyl, tert.-Pentyl, neo-Pentyl, n-Hexyl, n-Heptyl, n-Octyl, n-Nonyl, n-Decyl, n-Undecyl, n-Dodecyl und Stearyl, besonders bevorzugt C₁-C₄-Alkyl wie Methyl, Ethyl, n-Propyl, i-Propyl, n-Butyl, i-Butyl, sek.-Butyl, tert.-Butyl;
C₃-C₈-Cycloalkyl wie Cyclopropyl, Cyclopentyl, Cyclohexyl, Cycloheptyl, Cyclooctyl, 4-Methylcyclohexyl, 4-Methoxy-cyclohexyl, 2,4,6-Trimethylcyclohexyl;
C₃-C₈-Cycloalkyl-C₁-C₅-alkyl wie Cyclopropylmethyl, Cyclopentylmethyl, Cyclohexylmethyl, Cyclopropylethyl, Cyclopentylethyl, Cyclohexylethyl, Cyclopropylpropyl, Cyclopentylpropyl, Cyclohexylpropyl, Cyclopentylbutyl, Cyclohexylbutyl, Cyclopentylpentyl, Cyclohexylpentyl, Cyclooctylpentyl;
Hydroxy-C₁-C₅-alkyl wie Hydroxymethyl, 2-Hydroxyethyl, 2-Hydroxypropyl, 3-Hydroxypropyl, 4-Hydroxybutyl, 5-Hydroxypentyl, 2,2-Dimethyl-3-hydroxypropyl;
Amino-C₁-C₅-alkyl wie Aminomethyl, 2-Aminoethyl, 3-Aminopropyl, 4-Aminobutyl, 5-Aminopentyl;
N-C₁-C₄-Alkyl-amino-C₁-C₅-alkyl wie N-Methylaminomethyl, 2-(N-Methylamino)ethyl, 3-(N-Methylamino)propyl, 4-(N-Methylamino)butyl, 5-(N-Methylamino)pentyl, N-Ethylaminomethyl,N-n-Propylaminomethyl, N-n-Butylaminomethyl;
N,N-Di-(C₁-C₄-Alkyl)amino-C₁-C₅-alkyl wie N,N-Dimethylaminomethyl, 2-(N,N-Dimethylamino)ethyl, 3-(N,N-Dimethylamino)propyl, 4-(N,N-Dimethylamino)butyl, 5-(N,N-Dimethylamino)pentyl, N,N-Diethylaminomethyl, N,N-Di(n-Propyl)aminomethyl, N,N-Di(i-Propyl)aminomethyl, N,N-Di-(n-Butyl)aminomethyl, N-Ethyl-N-methyl-aminomethyl, N-Methyl-N-propyl-aminomethyl;
C₆-C₁₈-Aryl wie Phenyl, Naphthyl, Anthracenyl, Phenantrenyl, Azulenyl, Biphenylenyl, Triphenylenyl, bevorzugt Phenyl, wobei die Arylreste bis zu drei der unter R¹¹ genannten Gruppen tragen können;
C₆-C₁₈-Aryl-C₁-C₄-alkyl, bevorzugt Phenyl-C₁-C₄-alkyl wie Benzyl, 2-Phenylethyl, 3-Phenylpropyl, 4-Phenylbutyl, besonders bevorzugt Benzyl, 2-Phenylethyl, 3-Phenylpropyl, wobei die Arylgruppen bis zu drei der unter R¹¹ genannten Gruppen tragen können;
- R²,R³: C₁-C₁₈-Alkyl wie bei R¹ genannt, darunter besonders bevorzugt C₁-C₄-Alkyl wie Methyl, Ethyl, n-Propyl, i-Propyl, n-Butyl, sek.-Butyl, i-Butyl, tert.-Butyl;
C₃-C₈-Cycloalkyl wie Cyclopropyl, Cyclopentyl, Cyclohexyl, Cycloheptyl, Cyclooctyl, 4-Methylcyclohexyl, 2,4,6-Trimethylcyclohexyl;
C₆-C₁₈-Aryl wie bei R¹ genannt, vorzugsweise Phenyl, welches bis zu drei der unter R¹¹ genannten Gruppen tragen kann;
C₆-C₁₈-Aryl-C₁-C₄-alkyl wie bei R¹ genannt, vorzugsweise phenyl-C₁-C₄-alkyl, besonders bevorzugt Benzyl, 2-Phenylethyl, 3-Phenylpropyl, wobei die Phenylgruppe bis zu drei der unter R¹¹ genannten Gruppen tragen kann;
- R¹¹: Halogen wie Fluor, Chlor, Brom und Iod, C₁-C₂₂-Alkyl wie Methyl, Ethyl, n-Propyl, i-Propyl, n-Butyl, sek.-Butyl, i-Butyl, tert.-Butyl, n-Pentyl, i-Pentyl, sek.-Pentyl, tert.-Pentyl, neo-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosyl, n-Heneicosyl und n-Docosyl, vorzugsweise C₁-C₁₂-Alkyl wie Methyl, Ethyl, n-Propyl, i-Propyl, n-Butyl, sek.-Butyl, i-Butyl, tert.-Butyl, n-Pentyl, i-Pentyl, sek.-Pentyl, tert.-Pentyl, neo-Pentyl, n-Hexyl, n-Heptyl, n-Octyl, n-Nonyl, n-Decyl, n-Undecyl, n-Dodecyl und Stearyl, besonders bevorzugt C₁-C₄-Alkyl wie Methyl, Ethyl, n-Propyl, i-Propyl, n-Butyl, i-Butyl, sek.-Butyl, tert.-Butyl; C₁-C₄-Alkoxy wie Methoxy, Ethoxy, n-Propoxy und n-Butoxy, Carboxy, C₁-C₄-Alkoxycarbonyl wie Methoxycarbonyl, Ethoxycarbonyl, n-Propoxycarbonyl und n-Butoxycarbonyl, Aminocarbonyl, C₁-C₄-Alkylaminocarbonyl wie Methylaminocarbonyl, Ethylaminocarbonyl, n-Propylaminocarbonyl und n-Butylaminocarbonyl, Di-(C₁-C₄-Alkyl)aminocarbonyl wie Dimethylaminocarbonyl, Diethylaminocarbonyl, Di-(n-Propyl)aminocarbonyl und Di-(n-Butyl)aminocarbonyl, Nitrilo, Nitro, Amino, C₁-C₄-Alkylamino wie Methylamino, Ethylamino, n-Propylamino und n-Butylamino, Di-(C₁-C₄-alkyl)amino wie Dimethylamino, Diethylamino, Di-(n-Propyl)amino und Di-(n-Butyl)amino.

Dabei sind Verbindungen I ausgenommen, in denen A -COO-n-Butyl bedeutet, wenn B für -COO-C₁- bis C₂ -Alkyl steht und Verbindungen I, in denen A für -COO-Methyl steht, wenn B -COO-Ethyl bedeutet.

Bevorzugte Oxadimethacrylverbindungen I finden sich in der Tabelle I.

Oxamethylenverbindungen Ia, die die erfindungsgemäßen Verbindungen I sowie die ausgenommenen Verbindungen umfassen sind auf verschiedenen Wegen zugänglich.

Bei der ersten Variante des Verfahrens zur Herstellung der Oxadimethacrylverbindungen Ia ausgehend von einer Mischung der Acrylverbindungen IIa und IIb, H₂C=C(A)H und H₂C=C(B)H, geht man so vor, daß man zunächst die Acrylverbindungen II mit Formaldehyd oder Formaldehyd-liefernden Verbindungen in Gegenwart mindestens eines tertiären Amins und vorzugsweise mindestens eines Stabilisators, insbesondere eines polymerisationsinhibitors, zur Reaktion bringt. Gleichzeitig leitet man Sauerstoff oder ein Gemisch von Sauerstoff und einem oder mehreren nicht reaktiven Gasen über oder durch das Reaktionsgemisch. Das erhaltene Reaktionsgemisch enthält dann im wesentlichen die beiden Alkohole IIIa und IIIb, H₂C=C(A)CH₂OH und H₂C=C(B)CH₂OH.

Man kann nun das Reaktionsgemisch ohne Isolierung der Alkohole weiter umsetzen oder die Alkohole nach bekannten Methoden isolieren und dann weiter umsetzen. Praktisch vorteilhafter ist die Durchführung der Reaktion ohne Isolierung der Alkohole.

Im zweiten Teil der Reaktion erhitzt man sodann das die Alkohole IIIa und IIIb enthaltende Reaktionsgemisch, oder die isolierten Alkohole IIIa und IIIb, in Gegenwart mindestens eines tertiären Amins und mindestens eines Stabilisators, wobei man Sauerstoff, oder ein Sauerstoff enthaltendes Gasgemisch (enthaltend weitere, nicht reaktive Gase), über oder durch die Reaktionsmischung leitet. Hierbei erhält man ein Reaktionsgemisch enthaltend die entsprechenden Oxadimethacrylverbindung Ia, die man nach üblichen Methoden wie Chromatographie, Kristallisation oder Extraktion isolieren kann.

Die für das erfindungsgemäße Verfahren benötigten Acrylverbindungen II sind entweder käuflich oder man erhält sie beispielsweise durch Veresterung, Umesterung, Amidierung oder Aminolyse nach an sich bekannten Methoden (s. H.Rauch-Puntigam et al., Chemie, Physik und Technologie der Kunststoffe, Bd. 9, Springer Verlag, Berlin, 1967) aus den entsprechenden leicht zugänglichen Acrylvorstufen wie Acrylsäure und deren bekannte Derivate.

Den Formaldehyd kann man gasförmig, flüssig, beispielsweise als wäßrige Lösung wie Formalin oder in Form einer alkoholischen Lösung, oder in fester Form, zum Beispiel als para-Formaldehyd, Trioxan, Tetroxocan oder als Halbacetal einsetzen.

Des weiteren setzt man ein tertiäres Amin oder eine Mischung unterschiedlicher tertiärer Amine ein, wobei der Einsatz nur eines tertiären Amins bevorzugt ist. Als tertiäre Amine kommen offenkettige aliphatische oder cyclische tertiäre Amine in Betracht wie Trimethylamin, Triethylamin, Tri-n-propylamin, Triisopropylamin, Tri-n-butylamin, Triisobutylamin, Tri-n-pentylamin, Methyldiisopropylamin, N,N-Diethylisopropylamin, N,N-Dimethylethylamin, N,N-Dimethylisopropylamin, Tri-2-ethylhexylamin, N-Methyldiethylamin, N,N-Dimethyl-n-propylamin, N,N-Dimethyl-n-butylamin, N,N-Dimethylisobutylamin, N,N-Dimethyl-(2-ethylhexyl)amin, N,N-Diisopropyl-(2-ethylhexyl)amin, N,N-Di-n-butyl-(2-ethylhexyl)amin, N-Methyl-di-(2-ethylhexyl)amin, N-n-butyl-(2-ethylhexyl)amin, N-Isobutyl-di-(2-ethylhexyl)amin, Chinuclidin und 1,4-Diazabicyclo[2.2.2]octan (DAECO®), bevorzugt Chinuclidin und DABCO® besonders bevorzugt DABCO®.

Als Stabilisatoren verwendet man in der Regel die üblichen Polymerisationsinhibitoren wie Hydrochinon, Hydrochinonmonomethylether, p-Benzochinon, Phenol, 2,6-Dimethylphenol, 2,6-Di-tert.-Butylphenol, Methylenblau, Diphenylamin, Cu-(II)-oleat, Fe-(III)-acetylacetonat, Brenzkatechin, bevorzugt Hydrochinonmonomethylether und Hydrochinonmonoethylether.

Den Sauerstoff kann man in reiner Form oder in Form eines Gemisches mit nicht reaktiven Gasen, bevorzugt Luft, über oder durch das Reaktionsgemisch leiten.

In der ersten Stufe der Umsetzung, die zur Bildung der Alkoholverbindungen IIIa und IIIb führt, setzt man die Acrylverbindungen IIa und IIb und den Formaldehyd im allgemeinen im Molverhältnis Summe der Acrylverbindungen zu Formaldehyd von 1:1 bis 8:1, vorzugsweise von 1,0:1 bis 2,5:1, ein.

Das tertiäre Amin verwendet man hierbei vorzugsweise im Molverhältnis Formaldehyd zu Amin von 1:1 bis 200:1, bevorzugt 2:1 bis 100:1, besonders bevorzugt 4:1 bis 50:1.

Den Stabilisator setzt man in der Regel in Mengen von 10 bis 1000 mg pro kg der Acrylverbindungen ein.

Die Menge des Sauerstoffs liegt in der Regel im Bereich von 0,01 bis 100, bevorzugt von 0,1 bis 20 l/h pro kg der Acrylverbindungen. Verwendet man Luft als Sauerstofflieferanten, so wählt man die Gasmenge im allgemeinen im Bereich von 0,01 bis 1000, bevorzugt von 1 bis 250 l/h pro kg der Acrylverbindungen.

Im allgemeinen arbeitet man bei Temperaturen von 10 bis 100°C, bevorzugt von 40 bis 80°C, besonders bevorzugt von 60 bis 75°C. Des weiteren führt man die Reaktion in der Regel unter Atmosphärendruck durch. Sie kann jedoch auch bei vermindertem oder erhöhtem Druck, vorzugsweise im Bereich von 80 bis 250 kPa, vorgenommen werden. Ein Arbeiten unter Druck ist vor allem dann angezeigt, wenn man die Umsetzung bei Temperaturen oberhalb von 80°C vornimmt.

Des weiteren führt man die Reaktion in der Regel ohne Lösungsmittel durch. Jedoch kann man die Umsetzung auch in Gegenwart eines geeigneten Lösungsmittels wie eines C₅-C₈-Alkans, bevorzugt n-Pentan, n-Hexan, n-Heptan, n-Octan, i-Octan, eines Carbonsäureesters wie Acetylacetat, sowie eines aromatischen Lösungsmittels wie Benzol, Toluol und Xylole, besonders bevorzugt n-Hexan, i-Octan und Toluol, oder deren Mischungen, durchführen.

Die Reaktionszeit hängt hauptsächlich von der Reaktionstemperatur ab. Sie liegt im allgemeinen im Bereich von 1 bis 6 h.

Die bei dieser Reaktion gebildeten Alkohole IIIa und IIIb kann man nach den üblichen Aufarbeitungsmethoden wie Destillation oder Chromatographie isolieren.

In der zweiten Stufe des erfindungsgemäßen Verfahrens setzt man die nach der ersten Stufe erhaltene Mischung der isolierten Alkohole IIIa und IIIb, oder das diese Alkohole enthaltende Reaktionsgemisch in Gegenwart mindestens eines tertiären Amins und vorzugsweise mindestens eines Stabilisators unter Erhitzen zur Oxadimethacrylverbindung Ia um, wobei man gleichzeitig Sauerstoff oder ein Sauerstoff enthaltendes Gasgemisch, enthaltend weitere, nicht reaktive Gase, über oder durch die Reaktionsmischung leitet.

Art und Menge des Amins, des Stabilisators sowie des Lösungsmittels entsprechen denen, die bei der Reaktion der Acrylverbindungen II zu dem die Alkohole IIIa und IIIb enthaltenden Reaktionsgemisch bereits genannt wurden. Die Menge des Sauerstoffs liegt in der Regel im Bereich von 0,01 bis 1000, bevorzugt von 0,1 bis 50 l/h pro kg der Alkoholverbindungen IIIa und IIIb. Verwendet man Luft als Sauerstofflieferanten, so wählt man die Gasmenge im allgemeinen im Bereich von 0,1 bis 1000, bevorzugt von 1 bis 500 l/h pro kg der Alkoholverbindung IIIa und IIIb.

Die Umsetzung in der zweiten Stufe nimmt man im allgemeinen bei einer Temperatur im Bereich von 100 bis 200°C, bevorzugt von 100 bis 150°C, und bei einem Druck, der in der Regel im Bereich von 70 bis 300 kPa liegt, vor, bevorzugt arbeitet man jedoch unter Atmosphärendruck.

Das während der Reaktion anfallende Reaktionswasser kann man in der Regel durch Destillation, bevorzugt durch Rektifikation, aus dem Reaktionsgemisch entfernen.

Zweckmäßig kann man dabei dem Reaktionsgemisch ein Schleppmittel zusetzen. Hierfür eignen sich beispielsweise aliphatische, cycloaliphatische und aromatische Kohlenwasserstoffe sowie Carbonsäureester wie n-Hexan, n-Heptan, i-Octan, Benzol, Toluol, Xylol, Cyclohexan, Essigsäureethylester, oder gegebenenfalls die vor der Reaktion nicht abgetrennten Acrylverbindungen IIa und IIb. Den Siedepunkt der Schleppmittel wählt man im allgemeinen im Bereich zwischen 80 und 200°C.

Die Reaktionszeit ist von den üblichen Parametern wie Temperatur, Druck und den Mengen der Ausgangsstoffe abhängig und liegt in der Regel im Bereich von 4 bis 12 h.

Besonders vorteilhaft ist es, daß man das Reaktionsgemisch, das man bei der Herstellung der Alkohole IIIa und IIIb ausgehend von den Acrylverbindungen IIa und IIb nach dem weiter oben beschriebenen Verfahren erhält, ohne weitere Aufarbeitung zur Weiterumsetzung zu den entsprechenden Oxadimethacrylverbindungen Ia einsetzen kann.

Hierzu führt man die Reaktion zweckmäßig so durch, daß man nach dem oben beschriebenen erfindungsgemäßen Verfahren ausgehend von den Acrylverbindungen IIa und IIb zunächst die Alkohole IIIa und IIIb herstellt (erste Stufe), und dann das Reaktionsgemisch, ohne die Alkohole IIIa und IIIb zu isolieren, unter den Bedingungen weitererhitzt, wie sie bei der Herstellung der Oxadimethacrylverbindungen Ia (zweite Stufe) ausgehend von den Alkoholen IIa und IIb beschrieben wurden.

Die beim zweistufigen Verfahren im Reaktionsgemisch nach der Bildung der Alkohole IIIa und IIIb in der Regel im Überschuß noch vorhandenen Acrylverbindungen IIa und IIb, können jedoch auch vor der Weiterreaktion zur Oxadimethacrylverbindung Ia abgetrennt werden, beispielsweise durch Destillation. Dies kann jedoch auch nach der Reaktion zur Oxadimethacrylverbindung Ia erfolgen.

Man kann die unsymmetrischen Oxadimethacrylverbindungen Ia auch ausgehend von nur einer Acrylverbindung IIa herstellen (Variante B), indem man im Prinzip genauso vorgeht wie es bei der Herstellung der Alkohole IIIa und IIIb, ausgehend von einer Mischung der Acrylverbindungen IIa und IIb, beschrieben wurde (Variante A, erste Stufe). Auch hier kann man nach der Umsetzung der Acrylverbindung IIa mit Formaldehyd den entstandenen Alkohol IIIa isolieren oder im Reaktionsgemisch belassen. Im Unterschied zum ersten Verfahren, gibt man aber nach der Bildung des Alkohols IIIa einen weiteren Alkohol IIIb, der beispielsweise nach dem gleichen Verfahren oder auf einem anderem Wege gewonnen wurde, zum isolierten Alkohol IIIa oder zum den Alkohol IIIa enthaltenden Reaktionsgemisch hinzu. Anschließend verfährt man analog zur zweiten Stufe des ersten Verfahrens (A), indem man die Reaktionsmischung, enthaltend die Alkohole IIIa und IIIb, in Gegenwart mindestens eines tertiären Amins und vorzugsweise mindestens eines Stabilisators erhitzt, und gleichzeitig Sauerstoff oder ein Sauerstoff enthaltendes Gasgemisch, enthaltend weitere, nicht reaktive Gase, über oder durch die Reaktionsmischung leitet.

Art und Menge der Einsatzstoffe sowie die Versuchsparameter wählt man analog zu der bereits beschriebenen ersten bzw. zweiten Stufe des ersten Verfahrens (Variante A).

Es versteht sich von selbst, daß man Oxadimethacrylverbindungen Ia auch direkt von den Alkoholen IIIa und IIIb ausgehend herstellen kann (Variante C). Die Vorgehensweise entspricht der zweiten Stufe des ersten Verfahrens (A), so daß sie hier nicht wiederholt zu werden braucht.

Art und Menge der Einsatzstoffe sowie die Versuchsparameter wählt man analog zu der bereits beschriebenen zweiten Stufe des ersten Verfahrens (Variante A).

Selbstverständlich ist es auch möglich mehr als zwei Acrylverbindungen des Typs II bzw. Alkoholverbindungen des Typs III einzusetzen. Jedoch ist es im allgemeinen zweckmäßiger nur von jeweils zwei dieser Verbindungen auszugehen, um Reaktionsgemische, enthaltend mehrere Oxadimethacrylverbindungen des Typs I, zu vermeiden.

Ausgehend von Oxadimethacrylverbindungen der allgemeinen Formel I''

CH₂=C(E)CH₂-O-CH₂C(COQ)=CH₂ I''

in der E -COOR⁴ oder -CONR⁵R⁶, und Q -OR¹⁰ oder Halogen wie Chlor oder Brom bedeutet, wobei R¹⁰ die gleiche Bedeutung wie R¹ hat, mit der Maßgabe, daß R¹⁰ ≠ R⁷ ist, kann man Oxadimethacrylverbindungen der allgemeinen Formel I'

CH₂=C(E)CH₂-O-CH₂C(F)=CH₂ I'

in der F -COOR⁷ oder -CONR⁸R⁹ bedeutet, wobei R⁴ und R⁷ die gleiche Bedeutung wie R¹, R⁵ und R⁸ die gleiche Bedeutung wie R², und R⁶ und R⁹ die gleiche Bedeutung wie R³ haben, mit der Maßgabe, daß R⁴ ≠ R⁷, R⁵ ≠ R⁸ und R⁶ ≠ R⁹ sind, herstellen, indem man die Oxidimethacrylverbindungen I'' mit einem Alkohol, R⁷OH, oder einem Amin, HNR⁸R⁹, nach an sich bekannten Methoden umsetzt (s. Houben-Weyl, Methoden der organischen Chemie, Bd. VIII/III, S. 503 ff und S. 647 ff, 1952).

Oxadimethacrylverbindungen I'', in denen Q = -OR¹⁰ bedeutet, kann man nach den Verfahren wie sie weiter oben beschrieben wurden erhalten. Bei Symmetrischen Verbindungen, d.h. in den Fällen, in denen E = -COOR⁴ und Q = -OR⁴ ist, setzt man dabei entsprechend nur eine Acrylverbindung II bzw. eine Alkoholverbindung III anstelle von zwei dieser Verbindungen als Ausgangsverbindung für die Oxadimethacrylverbindungen I'' ein.

Oxadimethacrylverbindungen I'', in denen Q = Halogen bedeutet, kann man aus den entsprechenden Esterverbindungen (Q = -COOR¹) erhalten, indem man die Estergruppe zunächst zur Carbonsäuregruppe hydrolysiert und anschließend zum Carbonsäurehalogenid, bevorzugt dem Carbonsäurechlorid, nach an sich bekannten Methoden (S. Rauch-Puntigam et al., Chemie, Physik und Technologie der Kunststoffe, Bd 9., S. 79, Springer Verlag, Berlin, 1967) umsetzt.

Die bei diesen Verfahren hergestellten Oxadimethacrylverbindungen I kann man nach den üblichen Aufarbeitungsmethoden wie Destillation, Kristallisation oder Chromatographie isolieren.

Die Oxadimethacrylverbindungen I kann man als Monomere, Comonomere oder als Vernetzer verwenden, wobei man die Polymerisation beispielsweise nach der in der US-A 4,889,948 beschriebenen Methode durchführen kann. Des weiteren kann man sie auch durch eine 1,6-intra-intermolekulare Cyclopolymerisation zu cyclischen Ethern nach Methoden wie sie beispielsweise in Polymer Preprints, 31(1), 1990, 503 beschrieben sind, umsetzen. Hierdurch kann man im Vergleich zu Poly (meth)acrylaten eine höhere Wärmeformbeständigkeit erreichen.

Ein Vorteil gegenüber symmetrischen Oxadimethacrylverbindungen (A = B) liegt darin, daß man durch Wahl unterschiedlicher Reste A und B bestimmte Polymereigenschaften ohne Einbuße der Wärmeformbeständigkeit gezielt einstellen kann. So kann man beispielsweise den Brechungsindex in einem Oxadimethacryl-Polymer unter Beibehaltung einer guten Bewitterungsbeständigkeit dadurch erhöhen, daß man für den Rest A = - COOR¹ eine kurzkettige Alkylgruppe wie Methyl für R¹ wählt, während man für den Rest B = -COOR¹ einen aromatischen Rest wie Phenyl für R¹ wählt. Natürlich kann man auch zur weiteren Optimierung Copolymerisate herstellen.

Für die Verwendung als Beschichtungsmaterial oder zum Abmischen mit anderen Polymeren wie Polyamiden kann man bevorzugt solche Oxadimethacrylverbindungen Ia einsetzen, in denen A eine Estergruppe und B eine Amid- oder die Carbonsäuregruppe bedeuten.

Für die Herstellung wasserlöslicher Polymere kann man Oxadimethacrylverbindungen Ia mit einer Carbonsäuregruppe (A) und einer Carboxamidgruppe (B) verwenden.

Eine Verbesserung der Substrathaftung und der Verklebungsfähigkeit kann man min solchen Oxadimethacrylverbindungen I erreichen, in denen mindestens eine Gruppe (A und/oder B) einen quaternisierten Aminoalkylesterrest bedeutet.

### Beispiele

### Beispiel 1

### Darstellung von 2,2'[Oxybis(methylen)]-2-propensäure-ethylester-2'-propensäure-methylester nach dem erfindungsgemäßen Verfahren

Eine Mischung aus 430 g (5 mol) Methylacrylat, 500 g (5 mol) Ethylacrylat, 135 g (4,5 mol) para-Formaldehyd, 56 g (0,5 mol) DABCO® (1,4-Diazobicyclo-[2.2.2]-octan) und 200 mg Hydrochinonmonomethylether wurde während 3 h auf 75°C erhitzt, wobei gleichzeitig 10 l/h Luft durch die Mischung geleitet wurden. Dann wurde unter weiterem Erhitzen überschüssiges Methylacrylat und Ethylacrylat soweit abdestilliert, bis die Temperatur des Reaktionsgemisches im Sumpf 140°C betrug. Anschließend wurde das bei der Reaktion entstandene Reaktionswasser während 8 h bei 140°C abdestilliert, wobei als azeotropes Schleppmittel Isooctan diente. Danach erhielt man durch präparative Säulenchromatographie des Destillationsrückstandes an Silikagel mit Ethylacetat/Hexan (20/80) als Elutionsmittel
39 g 2,2'[Oxybis(methylen)]bis-2-propensäure-dimethylester
77 g (15,0 %) 2,2'[Oxybis(methylen)]-2-propensäure-ethylester-2'-propen-säure-methylester
53 g 2,2'[Oxybis(methylen)]bis-2-propensäure-diethylester.

### Beispiel 2

### Darstellung von 2,2'[Oxybis(methylen)]-2-propensäure-cyclohexylester-2'-propensäure-isopropylester (erfindungsgemäßes Produkt)

Eine Mischung aus 308 g (2 mol) Cylohexylacrylat, 228 g (2 mol) Isopropylacrylat, 60 g (2 mol) para-Formaldehyd, 22,4 g (0,2 mol) DABCO® und 100 mg Hydrochinonmonomethylether wurde während 6 h auf 75°C erhitzt, wobei gleichzeitig 10 l/h Luft durch die Mischung geleitet wurden. Dann wurde unter weiterem Erhitzen überschüssiges Cyclohexylacrylat und Isopropylacrylat soweit abdestilliert, bis die Temperatur des Reaktionsgemisches während 14 h im Sumpf auf 125°C stieg. Danach erhielt man durch präparative Säulenchromatographie des Destillationsrückstandes an Silikagel mit Ethylacetat/Hexan (20/80) als Elutionsmittel
51 g 2,2'[Oxybis(methylen)]bis-2-propensäure-isopropylester,
68 g (11,0 %) 2,2'[Oxybis(methylen)]-2-propensäure-cyclohexylester-2'-propensaure-isopropylester und
86 g 2,2'[Oxybis(methylen)]bis-2-propensäure-cyclohexylester.

Die Produktcharakterisierung erfolgte durch Elementaranalyse und ¹³C- und ¹H-NMR-spektroskopische Versuche (s. Tabelle 1).

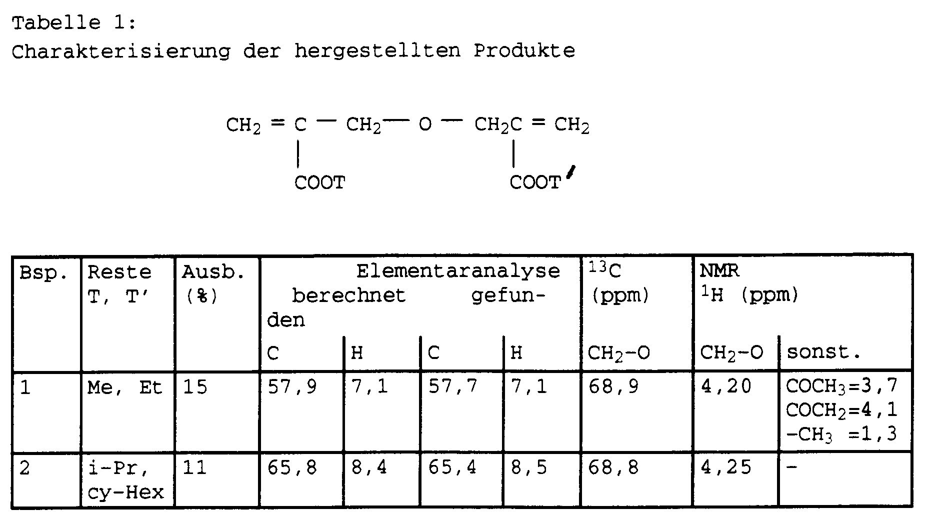

## Patentansprüche

1. Oxadimethacrylverbindungen der allgemeinen Formel I
CH₂=C(A)CH₂-O-CH₂C(B)=CH₂ I
in der A und B ausgewählt sind aus der Gruppe aus -COOR¹, -COR¹, -CONR²R³ und -CN, mit der Maßgabe, daß A ≠ B ist, und R¹, R² und R³ folgende Bedeutung haben:
R¹ = H, C₁-C₁₈-Alkyl, C₃-C₈-Cycloalkyl, C₃-C₈-Cycloalkyl-C₁-C₅-alkyl, wobei die Cycloalkyl-Ringe bis zu dreifach mit C₁-C₄-Alkyl oder C₁-C₄-Alkoxy-Gruppen substituiert sein können, Hydroxy-C₁-C₅-alkyl, Amino-C₁-C₅-alkyl, N-C₁-C₄-Alkyl-amino-C₁-C₅-alkyl, N,N-Di-(C₁-C₄-Alkyl)-amino-C₁-C₅-alkyl, C₆-C₁₈-Aryl, C₆-C₁₈-Aryl-C₁-C₄-alkyl, wobei die Arylgruppen bis zu drei der folgenden Gruppen tragen können: Halogen, C₁-C₂₂-Alkyl, C₁-C₄-Alkoxy, Carboxy, C₁-C₄-Alkoxycarbonyl, Aminocarbonyl, C₁-C₄-Alkylaminocarbonyl, Di-(C₁-C₄-Alkyl)aminocarbonyl, Nitrilo, Nitro, Amino, C₁-C₄-Alkylamino, Di-(C₁-C₄-Alkyl)amino;
R²,R³ = H, C₁-C₁₈-Alkyl, C₃-C₈-Cycloalkyl, C₃-C₆-Cycloalkyl-C₁-C₅-alkyl, wobei die Cycloalkyl-Ringe bis zu dreifach mit C₁-C₄-Alkyl- oder C₁-C₄-Alkoxy-Gruppen substituiert sein können, C₆-C₁₈-Aryl, C₆-C₁₈-Aryl-C₁-C₄-alkyl, wobei die Arylgruppen bis zu drei der folgenden Gruppen tragen können: Halogen, C₁-C₂₂-Alkyl, C₁-C₄-Alkoxy, Carboxy, C₁-C₄-Alkoxycarbonyl, Aminocarbonyl, C₁-C₄-Alkylaminocarbonyl, Di-(C₁-C₄-Alkyl)aminocarbonyl, Nitrilo, Nitro, Amino, C₁-C₄-Alkylamino, Di-(C₁-C₄-Alkyl)amino, ausgenommen Verbindungen I, in denen A -COO-n-Butyl bedeutet, wenn B für -COO-C₁- bis C₂-Alkyl steht und Verbindungen I, in denen A für -COO-Methyl steht, wenn B -COO-Ethyl bedeutet.

2. Verfahren zur Herstellung von Oxadimethacrylverbindungen der allgemeinen Formel Ia
CH₂=C(A)CH₂-O-CH₂C(B)=CH₂ Ia
in der A und B ausgewählt sind aus der Gruppe aus -COOR¹, -COR¹-, -CONR²R³ und -CN, mit der Maßgabe, daß A ≠ B und nicht -COOH bedeuten,und R¹, R² und R³ folgende Bedeutung haben:
R¹ = H, C₁-C₁₈-Alkyl, C₃-C₈-Cycloalkyl, C₃-C₈-Cycloalkyl-C₁-C₅-alkyl, wobei die Cycloalkyl-Ringe bis zu dreifach mit C₁-C₄-Alkyl- oder C₁-C₄-Alkoxy-Gruppen substituiert sein können, Hydroxy-C₁-C₅-alkyl, Amino-C₁-C₅-alkyl, N-C₁-C₄-Alkyl-amino-C₁-C₅-alkyl, N,N-Di-(C₁-C₄-Alkyl)-amino-C₁-C₅-alkyl, C₆-C₁₈-Aryl, C₆-C₁₈-Aryl-C₁-C₄-alkyl, wobei die Arylgruppen bis zu drei der folgenden Gruppen tragen können: Halogen, C₁-C₂₂-Alkyl, C₁-C₄-Alkoxy, Carboxy, C₁-C₄-Alkoxycarbonyl, Aminocarbonyl, C₁-C₄-Alkylaminocarbonyl, Di-(C₁-C₄-Alkyl)aminocarbonyl, Nitrilo, Nitro, Amino, C₁-C₄-Alkylamino, Di-(C₁-C₄-Alkyl)amino;
R²,R³ = H, C₁-C₁₈-Alkyl, C₃-C₈-Cycloalkyl, C₃-C₈-Cycloalkyl-C₁-C₅-alkyl, wobei die Cycloalkyl-Ringe bis zu dreifach mit C₁-C₄-Alkyl- oder C₁-C₄-Alkoxy-Gruppen substituiert sein können, C₆-C₁₈-Aryl, C₆-C₁₈-Aryl-C₁-C₄-alkyl, wobei die Arylgruppen bis zu drei der folgenden Gruppen tragen können: Halogen, C₁-C₂₂-Alkyl, C₁-C₄-Alkoxy, Carboxy, C₁-C₄-Alkoxycarbonyl, Aminocarbonyl, C₁-C₄-Alkylaminocarbonyl, Di-(C₁-C₄-Alkyl)aminocarbonyl, Nitrilo, Nitro, Amino, C₁-C₄-Alkylamino, Di-(C₁-C₄-Alkyl)amino
dadurch gekennzeichnet, daß man
A) eine Mischung aus zwei verschiedenen Acrylverbindungen der allgemeinen Formeln IIa und IIb
H₂C=C(A)H IIa
H₂C=C(B)H IIb
in Gegenwart von Sauerstoff und mindestens einem tertiären Amin mit Formaldehyd oder einer Formaldehyd-liefernden Verbindung zu den Alkoholen der allgemeinen Formeln
H₂C=C(A)CH₂OH IIIa
H₂C=C(B)CH₂OH IIIb
überführt, und dann entweder das diese Alkohole enthaltende Reaktionsgemisch, oder die aus dem Reaktionsgemisch isolierten Alkohole IIIa und IIIb unter Erhitzen in Gegenwart von Sauerstoff und mindestens einem tertiären Amin zur entsprechenden Oxadimethacrylverbindung I umsetzt, oder
B) eine Acrylverbindung IIa in Gegenwart von Sauerstoff und mindestens einem tertiären Amin mit Formaldehyd oder einer Formaldehyd-liefernden Verbindung zum Alkohol IIIa umsetzt, und dann den isolierten Alkohol IIIa oder das den nicht isolierten Alkohol IIIa enthaltende Reaktionsgemisch, mit einem weiteren, davon verschiedenen Alkohol IIIb unter Erhitzen in Gegenwart von Sauerstoff und mindestens einem tertiären Amin zur Oxadimethacrylverbindung I umsetzt, oder
C) eine Mischung aus zwei verschiedenen Alkoholen IIa und IIb in Gegenwart von Sauerstoff und mindestens einem tertiären Amin unter Erhitzen zur Oxadimethacrylverbindung I umsetzt.

3. Verfahren nach Anspruch 2, dadurch gekennzeichnet, daß die Menge Sauerstoff im Bereich von 0,01 bis 100 l/h pro kg Acrylverbindung oder 0,01 bis 1000 l/h pro kg Alkoholverbindung liegt.

4. Verfahren nach Anspruch 2 oder 3, dadurch gekennzeichnet, daß man die Umsetzung in Gegenwart von Hydrochinonmonomethylether durchführt.

5. Verfahren nach einem der Ansprüche 2 bis 4, dadurch gekennzeichnet, daß man die erste Reaktionsstufe bei Temperaturen von 40 bis 80°C und die zweite Reaktionsstufe bei Temperaturen von 100 bis 150°C durchführt.

6. Verfahren zur Herstellung von Oxadimethacrylverbindungen der allgemeinen Formel I'
CH₂=C(E)CH₂-O-CH₂C(F)=CH₂ I'
in der E -COOR⁴ oder -CONR⁵R⁶ und F -COOR⁷ oder -CONR⁸R⁹ bedeuten, wobei R⁴ und R⁷ die gleiche Bedeutung wie R¹, R⁵ und R⁸ die gleiche Bedeutung wie R², und R⁶ und R⁹ die gleiche Bedeutung wie R³ haben, mit der Maßgabe, daß R⁴ ≠ R⁷, R⁵ ≠ R⁸ und R⁶ ≠ R⁹ ist, dadurch gekennzeichnet, daß man Oxadimethacrylverbindungen der allgemeinen Formel I''
CH₂=C(E)CH₂-O-CH₂C(COQ)=CH₂ I''
in der Q -OR¹⁰ oder Halogen bedeutet, wobei R¹⁰ die gleiche Bedeutung wie R¹ hat, mit der Maßgabe, daß R¹⁰ ≠R⁷,
mit einem Alkohol, R⁷OH, oder einem Amin, HNR⁸R⁹, umsetzt.

7. Verwendung der Oxadimethacrylverbindungen Ia gemäß Anspruch 2 zur Herstellung von Polymeren.

8. Polymerisate, erhältlich aus Oxadimethacrylverbindungen Ia gemäß Anspruch 2 hergestellt.

## Claims

1. Oxadimethacrylics of the general formula I
CH₂=C(A)CH₂-O-CH₂C(B)=CH₂ I
where A and B are selected from the group consisting of -COOR¹, -COR¹, -CONR²R³ and -CN with the proviso that A ≠ B, and R¹, R² and R³ are each defined as follows:
R¹ is hydrogen, C₁-C₁₈-alkyl, C₃-C₈-cycloalkyl, C₃-C₈-cycloalxyl-C₁-C₅-alkyl, wherein the cycloalkyl rings may be C₁-C₄-alkyl- or C₁-C₄-alxoxy-monosubstitinted, -disubstituted or -trisubstituted, hydroxy-C₁-C₅-alkyl, amino-C₁-C₅-alkyl, N-C₁-C₄-alkylamino-C₁-C₅-alkyl, N,N-di(C₁-C₄-alkyl)amino-C₁-C₅-alkyl, C₆-C₁₈-aryl, C₆-C₁₈-aryl-C₁-C₄-alkyl, wherein the aryl groups may carry up to three of the following groups: halogen, C₁-C₂₂-alkyl, C₁-C₄-alkoxy, carboxyl, C₁-C₄-alkoxycarbonyl, aminocarbonyl, C₁-C₄-alkylaminocarbonyl,di(C₁-C₄-alkyl)aminocarbonyl, nitrilo, nitro, amino, C₁-C₄-alkylamino, di(C₁-C₄-alkyl)amino;
R² and R³ are each hydrogen, C₁-C₁₈-alkyl, C₃-C₈-cycloalkyl, C₃-C₈-cycloalkyl-C₁-C₅-alkyl, wherein the cycloalkyl rings may be C₁-C₄-alkyl- or C₁-C₄-alkoxy-monosubstitinted, -disubstituted or -trisubstituted, C₆-C₁₈-aryl, C₆-C₁₈-aryl-C₁-C₄-alkyl, wherein the aryl groups may carry up to three of the following groups: halogen, C₁-C₂₂-alkyl, C₁-C₄-alkoxy, carboxyl, C₁-C₄-alkoxycarbonyl, aminocarbonyl, C₁-C₄-alkylaminocarbonyl,di(C₁-C₄-alkyl)aminocarbonyl, nitrilo, nitro, amino, C₁-C₄-alkylamino, di(C₁-C₄-alkyl)amino, excluding compounds I in which A is -COO-n-butyl when B is -COO-C₁- to C₂-alkyl and compounds I in which A is -COO-methyl when B is -COO-ethyl.

2. A process for preparing oxadimethacrylics of the general formula Ia
CH₂=C(A)CH₂-O-CH₂C(B)=CH₂ Ia
where A and B are selected from the group consisting of -COOR¹, -COR¹, -CONR²R³ and -CN with the proviso that A ≠ B and is not -COOH, and R¹, R² and R³ are each defined as follows:
R¹ is hydrogen, C₁-C₁₈-alkyl, C₃-C₈-cycloalkyl, C₃-C₈-cycloalkyl-C₁-C₅-alkyl, wherein the cycloalkyl rings may be C₁-C₄-alkyl- or C₁-C₄-alkoxy-monosubstitinted, -disubstituted or -trisubstituted, hydroxyC₁-C₅-alkyl, amino-C₁-C₅-alkyl, N-C₁-C₄-alkylaminoC₁-C₅-alkyl, N,N-di(C₁-C₄-alkyl)amino-C₁-C₅-alkyl, C₆-C₁₈-aryl, C₆-C₁₈-aryl-C₁-C₄-alkyl, wherein the aryl groups may carry up to three of the following groups: halogen, C₁-C₂₂-alkyl, C₁-C₄-alkoxy, carboxyl, C₁-C₄-alkoxycarbonyl, aminocarbonyl, C₁-C₄-alkylaminocarbonyl,di(C₁-C₄-alkyl)aminocarbonyl, nitrilo, nitro, amino, C₁-C₄-alkylamino, di(C₁-C₄-alkyl)amino;
R² and R³ are each hydrogen, C₁-C₁₈-alkyl, C₃-C₈-cycloalkyl, C₃-C₈-cycloalkyl-C₁-C₅-alkyl, wherein the cycloalkyl rings may be C₁-C₄-alkyl- or C₁-C₄-alkoxy-monosinbstitinted, -disubstitinted or -trisubstituted, C₆-C₁₈-aryl, C₆-C₁₈-aryl-C₁-C₄-alkyl, wherein the aryl groups may carry up to three of the following groups: halogen, C₁-C₂₂-alkyl, C₁-C₄-alkoxy, carboxyl, C₁-C₄-alkoxycarbonyl, aminocarbonyl, C₁-C₄-alkylaminocarbonyl,di(C₁-C₄-alkyl)aminocarbonyl, nitrilo, nitro, amino, C₁-C₄-alkylamino, di(C₁-C₄-alkyl)amino,
which comprises
A) reacting a mixture of two different acrylics of the general formulae IIa and IIb
H₂C=C(A)H IIa
H₂C=C(B)H IIb
with formaldehyde or a formaldehyde donor compound in the presence of oxygen and of at least one tertiary amine to form the alcohols of the general formulae
H₂C=C(A)CH₂OH IIIa
H₂C=C(B)CH₂OH IIIb
and then converting either the reaction mixture containing these alcohols, or the isolated alcohols IIIa and IIIb, into the corresponding oxadimethacrylic I by heating in the presence of oxygen and of at least one tertiary amine, or
B) reacting an acrylic IIa with formaldehyde or a formaldehyde donor compound in the presence of oxygen and of at least one tertiary amine to form the alcohol IIIa and then reacting the isolated alcohol IIIa or the reaction mixture containing the nonisolated alcohol IIIa with a further, different alcohol IIIb by heating in the presence of oxygen and of at least one tertiary amine to form the oxadimethacrylic I, or
C) converting a mixture of two different alcohols IIIa and IIIb into the oxadimethacrylic I by heating in the presence of oxygen and of at least one tertiary amine.

3. A process as claimed in claim 2 wherein the amount of oxygen ranges from 0.01 to 100 l/h per kg of acrylic or from 0.01 to 1000 l/h per kg of alcohol.

4. A process as claimed in claim 2 or 3 wherein the reaction is carried out in the presence of hydroquinone monomethyl ether.

5. A process as claimed in any of claims 2 to 4 wherein the first reaction stage is carried out at from 40 to 80°C and the second reaction stage is carried out at from 100 to 150°C.

6. A process for preparing oxadimethacrylics of the general formula I'
CH₂=C(E)CH₂-O-CH₂C(F)=CH₂ I'
where E is -COOR⁴ or -CONR⁵R⁶ and F is -COOR⁷ or -CONR⁸R⁹ wherein R⁴ and R⁷ each have the same meaning as R¹, R⁵ and R⁸ each have the same meaning as R², and R⁶ and R⁹ each have the same meaning as R³, with the proviso that R⁴ ≠ R⁷, R⁵ ≠ R⁸ and R⁶ ≠ R⁹, which comprises reacting oxadimethacrylics of the general formula I''
CH₂=C(E)CH₂-O-CH₂C(COQ)=CH₂ I''
where Q is -OR¹⁰ or halogen, wherein R¹⁰ has the same meaning as R¹, with the proviso that R¹⁰ ≠ R⁷,
with an alcohol, R⁷OH, or an amine, HNR⁸R⁹.

7. The use of oxadimethacrylics Ia as set forth in claim 2 for preparing polymers.

8. Addition polymers prepared as obtainable from oxadimethacrylics Ia as set forth in claim 2.

## Revendications

1. Composés oxadiméthacryliques de formule générale I
CH₂=C(A)CH₂-O-CH₂C(B)=CH₂ I
dans laquelle A et B sont choisis dans le groupe de -COOR¹, de -COR¹, de -CONR²R³ et de -CN, étant spécifié que A est différent de B et que R¹, R² et R³ ont les significations suivantes:
R¹ Atome d'hydrogène, groupement alkyle en C₁-C₁₈, cycloalkyle en C₃-C₈, (cycloalkyl en C₃-C₈)alkyle en C₁-C₅, les noyaux cycloalkyle pouvant être substitués jusqu'à trois fois par des restes alkyle en C₁-C₄ ou alcoxy en C₁-C₄, hydroxyalkyle en C₁-C₅, aminoalkyle en C₁-C₅, N-(alkyl en C₁-C₄)aminoalkyle en C₁-C₅, N,N-di(alkyl en C₁-C₄)aminoalkyle en C₁-C₅, aryle en C₆-C₁₈, (aryl en C₆-C₁₈)alkyle en C₁-C₄, les groupements aryle pouvant porter jusqu'à trois des restes suivants: atomes d'halogène, restes alkyle en C₁-C₂₂, alcoxy en C₁-C₄, carboxy, (alcoxy en C₁-C₄)carbonyle, aminocarbonyle, (alkyl en C₁-C₄)aminocarbonyle, di(alkyl en C₁-C₄)aminocarbonyle, nitrilo, nitro, amino, (alkyl en C₁-C₄)amino, di(alkyl en C₁-C₄)amino;
R²,R³ Atomes d'hydrogène, groupements alkyle en C₁-C₁₈, cycloalkyle en C₃-C₈, (cycloalkyl en C₃-C₈)alkyle en C₁-C₅, les noyaux cycloalkyle pouvant être substitués jusqu'a trois fois par des restes alkyle en C₁-C₄ ou alcoxy en C₁-C₄, aryle en C₆-C₁₈, (aryl en C₆-C₁₈)alkyle en C₁-C₄, les groupements aryle pouvant porter jusqu'à trois des restes suivants: atomes d'halogène, restes alkyle en C₁-C₂₂, alcoxy en C₁-C₄, carboxy, (alcoxy en C₁-C₄)carbonyle, aminocarbonyle, (alkyl en C₁-C₄)aminocarbonyle, di(alkyl en C₁-C₄)aminocarbonyle, nitrilo, nitro, amino, (alkyl en C₁-C₄)amino, di(alkyl en C₁-C₄)amino,
à l'exception des composés I dans lesquels A représente le groupement -COO-n-butyle lorsque B est mis pour un groupement -COO-alkyle en C₁-C₂, et des composés I dans lesquels A est mis pour le groupement -COO-méthyle lorsque B représente le groupement -COO-éthyle.

2. Procédé de préparation de composés oxadiméthacryliques de formule générale Ia
CH₂=C(A)CH₂-O-CH₂C(B)=CH₂ Ia
dans laquelle A et B sont choisis dans le groupe de -COOR¹, de -COR¹, de -CONR²R³ et de -CN, étant spécifié que A est différent de B et qu'ils ne représentent pas -COOH, et que R¹, R² et R³ ont les significations suivantes:
R¹ Atome d'hydrogène, groupement alkyle en C₁-C₁₈, cycloalkyle en C₃-C₈, (cycloalkyl en C₃-C₈)alkyle en C₁-C₅, les noyaux cycloalkyle pouvant être substitués jusqu'à trois fois par des restes alkyle en C₁-C₄ ou alcoxy en C₁-C₄, hydroxyalkyle en C₁-C₅, aminoalkyle en C₁-C₅, N-(alkyl en C₁-C₄)aminoalkyle en C₁-C₅, N,N-di(alkyl en C₁-C₄)aminoalkyle en C₁-C₅, aryle en C₆-C₁₈, (aryl en C₆-C₁₈(alkyle en C₁-C₄, les groupements aryle pouvant porter jusqu'à trois des restes suivants: atomes d'halogène, restes alkyle en C₁-C₂₂, alcoxy en C₁-C₄, carboxy, (alcoxy en C₁-C₄)carbonyle, aminocarbonyle, (alkyl en C₁-C₄)aminocarbonyle, di(alkyl en C₁-C₄)aminocarbonyle, nitrilo, nitro, amino, (alkyl en C₁-C₄)amino, di(alkyl en C₁-C₄)amino;
R²,R³ Atomes d'hydrogène, groupements alkyle en C₁-C₁₈, cyoloalkyle en C₃-C₈, (cycloalkyl en C₃-C₈)alkyle en C₁-C₅, les noyaux cycloalkyle pouvant être substitués jusqu'a trois fois par des restes alkyle en C₁-C₄ ou alcoxy en C₁-C₄, aryle en C₆-C₁₈, (aryl en C₆-C₁₈)alkyle en C₁-C₄, les groupements aryle pouvant porter jusqu'à trois des restes suivants: atomes d'halogène, restes alkyle en C₁-C₂₂, alcoxy en C₁-C₄, carboxy, (alcoxy en C₁-C₄)carbonyle, aminocarbonyle, (alkyl en C₁-C₄)aminocarbonyle, di(alkyl en C₁-C₄)aminocarbonyle, nitrilo, nitro, amino, (alkyl en C₁-C₄)amino, di(alkyl en C₁-C₄)amino,
caractérisé en ce que
A) l'on transforme un mélange de deux composés acryliques différents de formules générales IIa et IIb
H₂C=C(A)H IIa
H₂C=C(B)H IIb
en présence d'oxygène et d'au moins une amine tertiaire, avec du formaldéhyde ou un composé générateur de formaldéhyde, en les alcools de formules générales
H₂C=C(A)CH₂OH IIIa
H₂C=C(B)CH₂OH IIIb
puis on transforme, soit le mélange réactionnel contenant ces alcools, soit les alcools IIIa et IIIb isolés du mélange réactionnel, sous chauffage et en présence d'oxygène et d'au moins une amine tertiaire, en le composé oxadiméthacrylique I correspondant, ou bien
B) on transforme un composé acrylique IIa, en présence d'oxygène et d'au moins une amine tertiaire, avec du formaldéhyde ou un composé générateur de formaldéhyde, en l'alcool IIIa, puis on transforme l'alcool IIIa isolé ou le mélange réactionnel contenant l'alcool IIIa non isolé, avec un autre alcool IIIb différent de celui-ci, sous chauffage et en présence d'oxygène et d'au moins une amine tertiaire, en le composé oxadiméthacrylique I, ou bien
C) on transforme un mélange de deux alcools IIa et IIb différents, en présence d'oxygène et d' au moins une amine tertiaire, sous chauffage, en le composé oxadiméthacrylique I.

3. Procédé selon la revendication 2, caractérisé en ce que la quantité d'oxygéne se situe dans l'intervalle de 0,01 à 100 l/h par kg de composé acrylique ou de 0,01 à 1000 l/h par kg de composé alcool.

4. Procédé selon la revendication 2 ou 3, caractérisé en ce que l'on conduit la transformation en présence d'éther monométhylique d'hydroquinone.

5. Procédé selon l' une quelconque des revendications 2 à 4, caractérisé en ce que l'on mène la première étape de réaction à des températures de 40 à 80°C et la seconde étape de réaction à des températures de 100 à 150°C.

6. Procédé de préparation de composés oxadiméthacryliques de formule générale I'
CH₂=C(E)CH₂-O-CH₂C(F)=CH₂ I'
dans laquelle E représente -COOR⁴ ou -CONR⁵R⁶ et F représente -COOR⁷ ou -CONR⁸R⁹, R⁴ et R⁷ ayant la même signification que R¹, R⁵ et R⁸ la même signification que R², et R⁶ et R⁹ la même signification que R³, étant spécifié que R⁴ ≠ R⁷, R⁵ ≠ R⁸ et R⁶ ≠ R⁹, caractérisé en ce l'on fait réagir des composés oxadiméthacryliques de formule générale I''
CH₂=C(E)CH₂-O-CH₂C(COQ)=CH₂ I''
dans laquelle Q représente un groupement -OR¹⁰ ou un atome d'halogéne R¹⁰ ayant la même signification que R¹, étant spécifié que R¹⁰ ≠ R⁷,
avec un alcool R⁷OH ou une amine HNR⁸R⁹.

7. Utilisation des composés oxadiméthacryliques Ia selon la revendication 2 pour la préparation de polymères.

8. Polymères obtenus à partir de composés oxadiméthacryliques Ia préparés selon la revendication 2.
